Europäisches Patentamt

European Patent Office

Office européen des brevets

(19)

(11) **EP 0 558 424 B1**

(12)

# FASCICULE DE BREVET EUROPEEN

(45) Date de publication et mention
de la délivrance du brevet:
**04.06.1997 Bulletin 1997/23**

(51) Int Cl.6: **C07C 253/24**

(21) Numéro de dépôt: **93420042.9**

(22) Date de dépôt: **26.01.1993**

(54) **Procédé d'ammoxydation d'hydrocarbures saturés**

Prozess zur Ammoxidation von gesättigten Kohlenwasserstoffen

Process for the ammoxydation of saturated hydrocarbons

(84) Etats contractants désignés:
**BE DE ES FR GB IT NL PT**

(30) Priorité: **20.02.1992 FR 9202195**

(43) Date de publication de la demande:
**01.09.1993 Bulletin 1993/35**

(73) Titulaire: **RHONE-POULENC FIBER & RESIN
INTERMEDIATES
92405 Courbevoie (FR)**

(72) Inventeurs:
• **Blanchard, Gilbert
F-60330 Le Plessis Belleville (FR)**
• **Ferre, Gilbert
F-93190 Livry Gargan (FR)**

(74) Mandataire: **Vignally, Noel et al
RHONE-POULENC CHIMIE,
Direction de la Propriété Industrielle,
Centre de Recherches des Carrières,
B.P. 62
69192 Saint-Fons Cédex (FR)**

(56) Documents cités:
GB-A- 1 336 136          US-A- 3 927 007
US-A- 4 871 706          US-A- 5 008 427

Remarques:
Le dossier contient des informations techniques
présentées postérieurement au dépôt de la
demande et ne figurant pas dans le présent
fascicule.

## Description

La présente invention concerne un procédé d'ammoxydation d'hydrocarbures saturés, c'est-à-dire la conversion d'alcanes en un mélange renfermant des nitriles $\alpha,\beta$-insaturés.

Il est bien connu de l'homme de l'art que de très nombreuses propositions ont été formulées en ce qui concerne l'ammoxydation des oléfines et, en particulier celle du propylène. Toutefois, bien que les hydrocarbures saturés, plus largement disponibles, soient des matières premières plus intéressantes au plan économique, il est également bien connu qu'ils ne présentent pas une réactivité comparable dans ce type de réaction pour former notamment des nitriles $\alpha,\beta$-insaturés.

L'une des difficultés rencontrées dans l'ammoxydation des hydrocarbures saturés réside dans la nécessité de pouvoir disposer de catalyseurs susceptibles de déshydrogéner l'hydrocarbure saturé dans des conditions minimisant ou supprimant la combustion de l'ammoniac et/ou celle de l'hydrocarbure, tout en assurant une sélectivité raisonnable, soit en nitrile $\alpha,\beta$-insaturé (produit visé), par exemple en acrylonitrile au départ du propane, soit en produits valorisables (nitrile précité et oléfine), par exemple en acrylonitrile et propylène au départ du propane.

Il a déjà été proposé dans le brevet américain n° 3 365 482 d'ammoxyder notamment l'isobutane en méthacrylonitrile, sur un catalyseur à base de molybdène déposé sur de l'éta-alumine dopée par de l'antimoine, à 508°C, au départ d'un mélange gazeux renfermant de l'isobutane, de l'air, de l'ammoniac et de la vapeur d'eau (1,0/4,5/1,0/12,5) ; la sélectivité en méthacrylonitrile atteint 49 % pour un taux de conversion de l'isobutane de 22 %.

Au départ d'un mélange gazeux propane/air/ammoniac/vapeur d'eau (1,0/4,7/0,67/12,8), avec le même catalyseur et à 550°C, la sélectivité en acrylonitrile tombe à 15 % pour un taux de transformation du propane de 29 %.

Dans "Chemistry letters 1989 (pages 2173-2176)" des auteurs ont testés, pour l'ammoxydation du propane en phase vapeur, des oxydes métalliques multicomposants renfermant du molybdène et du bismuth et présentant une structure du type de celle de la scheelite. Il apparaît que, malgré les températures relativement modérées mises en oeuvre, la proportion de produits de combustion ($CO$, $CO_2$) est très élevée dans tous les cas (au moins 15 %) et que certaines compositions catalytiques testées sont très peu actives vis-à-vis de la réaction souhaitée, malgré leur mise en oeuvre dans des conditions se situant dans le domaine d'explosivité ou très proches dudit domaine.

Il est manifeste que la coproduction de grandes quantités de $CO$ et de $CO_2$ est indésirable à l'échelle industrielle.

Au surplus, l'utilisation de mélanges réactionnels se situant dans le domaine d'explosivité est d'autant moins souhaitable à l'échelle industrielle que l'on met en oeuvre le procédé en lit fixe.

Le brevet US-A-5 008 427 décrit un procédé d'ammoxydation de propane ou d'isobutane en mononitrile $\alpha,\beta$ insa-turé, par réaction en phase vapeur avec de l'oxygène et de l'ammoniac, en présence d'un catalyseur comportant du vanadium, de l'antimoine, de l'oxygène et du titane et/ou de l'étain et/ou du fer et/ou du chrome et/ou du gallium et éventuellement un ou plusieurs autres élements choisis parmi 23 métaux très divers. En dehors de la composition du catalyseur, les caractéristiques principales du procédé décrit dans ce document sont la température de calcination du catalyseur, qui doit être d'au moins 780°C, et les rapports molaires alcane/ammoniac (de 2,5 à 16) et alcane/oxygène (de 1 à 10).

Ce procédé permettrait d'obtenir une bonne productivité en nitrile insaturé avec un catalyseur ne s'agglomérant pas.

Cependant ce document, qui est donc essentiellement axé sur des conditions opératoires telles que température de calcination ou rapports des réactifs, ne permet pas de sélectionner plus particulièrement des compositions de phase active permettant une bonne sélectivité en nitrile insaturé.

Il demeure donc souhaitable de disposer d'un procédé d'ammoxydation d'alcanes, permettant d'obtenir avec une sélectivité appréciable un mélange de produits valorisables renfermant un nitrile $\alpha,\beta$-insaturé, en particulier de l'acrylonitrile, tout en diminuant les pertes de matière première par suite notamment de la formation d'oxydes de carbone.

Il serait également hautement souhaitable de disposer d'un tel procédé dans lequel le catalyseur solide serait relativement simple à préparer et actif en l'absence de promoteur halogéné et pour des mélanges de gaz ne se situant pas nécessairement dans le domaine d'explosivité.

La présente invention a donc pour objet un procédé d'ammoxydation d'alcanes en phase vapeur, en présence d'un catalyseur solide comportant au moins une phase active, caractérisé en ce que la dite phase active répond à la formule empirique suivante (I) :

$$V\,Sb_a M_b Ox \tag{I}$$

dans laquelle :

- a représente un nombre entier ou fractionnaire égal ou supérieur à 1,

- M représente un atome de fer et/ou de gallium et/ou d'indium,
- b représente un nombre entier ou fractionnaire égal ou supérieur à 0,5,
- x représente un nombre entier ou fractionnaire déterminé par le degré d'oxydation des autres éléments de la phase active.

Préférentiellement dans la formule (I) de la phase active décrite précédemment :

- le symbole a représente un nombre entier ou fractionnaire jusqu'à 20 et est de préférence compris entre 1 et 10,
- le symbole b représente un nombre entier ou fractionnaire jusqu'à 20 et est de préférence compris entre 1 et 10.

Parmi les métaux représentés par M dans la formule (I) de la phase active, on préfère généralement le fer qui permet d'obtenir d'excellentes sélectivités en nitrile insaturé et en hydrocarbure éthylénique, tout en ayant une bonne activité.

Selon la présente invention des hydrocarbures saturés acycliques ayant de 3 à 12 atomes de carbone par molécule sont mis à réagir en phase vapeur avec de l'ammoniac et de l'oxygène en présence d'un catalyseur dont la phase active vient d'être précisée.

Bien entendu, dans le cadre du présent procédé, on peut utiliser des gaz diluants, inertes dans les conditions réactionnelles tels que l'hélium, l'azote et l'argon. De même, de la vapeur d'eau peut être ajoutée au mélange gazeux réactionnel dans de larges limites. Ainsi le gaz réactif (hydrocarbure saturé, ammoniac, oxygène) peut être dilué par un diluant inerte et/ou par de la vapeur d'eau. Dans cet ensemble la teneur en vapeur d'eau peut varier dans de larges limites en particulier de 0 à 50 % et, de préférence entre 3 et 30 %. Pour une bonne mise en oeuvre du procédé selon l'invention, la teneur en gaz réactif sera d'au moins 3 % et de préférence d'au moins 20 %.

Au sein du gaz réactif les teneurs respectives en hydrocarbure saturé, ammoniac et oxygène peuvent varier dans de larges limites.

La teneur en hydrocarbure saturé dans le gaz réactif est de préférence comprise entre 5 et 70 %. Celle en ammoniac est de préférence comprise entre 3 et 50 % et celle en oxygène est de préférence comprise entre 3 et 45 %.

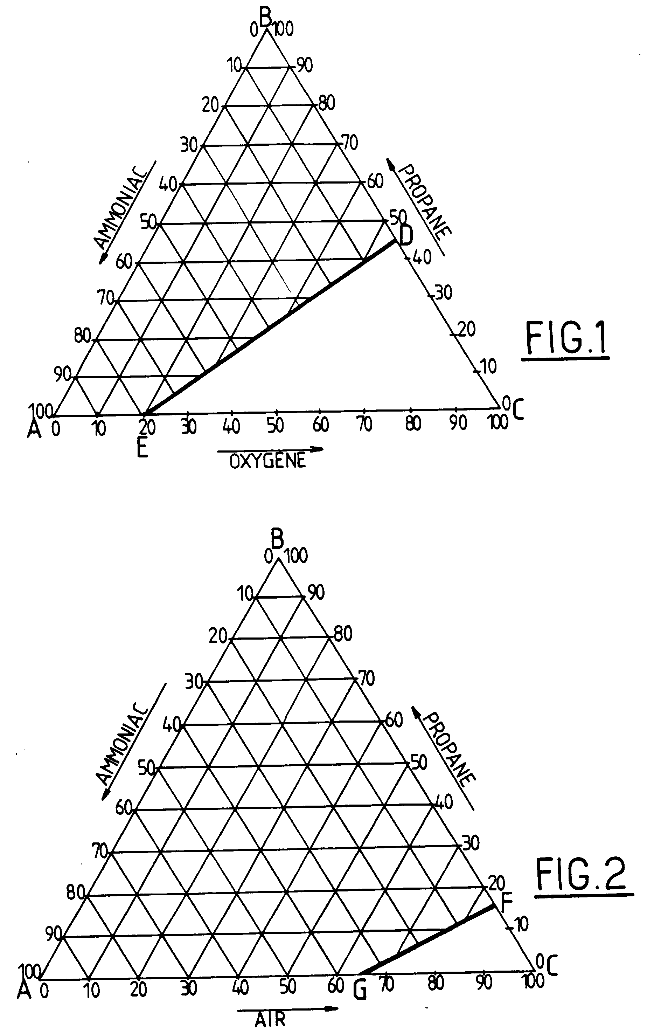

Pour une bonne mise en oeuvre du procédé selon l'invention la composition du mélange réactif sera en dehors du domaine d'explosivité. S'agissant de l'ammoxydation du propane en l'absence de diluant inerte, la composition (propane, oxygène, ammoniac) sera avantageusement choisie à l'intérieur du quadrilatère ABDE figurant au sein du diagramme ternaire ABC représenté sur la figure 1 annexée.

Sur ce diagramme ternaire le segment AB représente la teneur en ammoniac de 100 % à 0 % ; le segment BC représente la teneur en propane de 100 à 0 % ; le segment CA représente la teneur en oxygène de 100 à 0 %. Le point D, situé sur le segment BC, correspond à une teneur en propane de 45 % ; dans le binaire (propane-$O_2$) ; le point E situé sur le segment AC, correspond à une teneur en ammoniac de 79 % dans le binaire (ammoniac-$O_2$).

Le segment DE délimite le diagramme ternaire en deux parties : un triangle CDE à l'intérieur duquel se situe la zone d'explosivité (déterminée sous 1 bar et à 25°C) et un quadrilatère ABDE à l'intérieur duquel la composition du mélange gazeux réactif sera avantageusement choisie.

S'agissant de l'ammoxydation du propane en présence de gaz diluant(s) inerte(s) et/ou de vapeur d'eau, il conviendra de déterminer la composition du mélange ternaire (propane, oxygène et ammoniac) pour la situer sur le diagramme précité, lorsque le gaz diluant et/ou la vapeur d'eau est en faible proportion.

S'agissant de l'ammoxydation du propane au moyen de l'air comme source d'oxygène, la composition (propane, air et ammoniac) sera avantageusement choisie à l'intérieur du quadrilatère ABFG figurant au sein du diagramme ABC représenté sur la figure 2 annexée.

Sur ce second diagramme le segment AB représente la teneur en ammoniac de 100 % à 0 % ; le segment BC représente la teneur en propane de 100 à 0 % : le segment CA représente la teneur en air de 100 à 0 %. Le point F, situé sur le segment BC, correspond à une teneur en propane de 16 % dans le binaire (propane-air) ; le point G situé sur le segment AC, correspond à une teneur en ammoniac de 35 % dans le binaire (ammoniac-air).

Le segment FG délimite le diagramme ternaire en deux parties : un triangle CFG à l'intérieur duquel se situe la zone d'explosivité (déterminée sous 1 bar à 550°C) et un quadrilatère ABFG à l'intérieur duquel la composition du mélange gazeux réactif sera avantageusement choisie.

Ce second diagramme sera utilisé dans le cas où le mélange oxygène-gaz diluant correspond à une teneur en oxygène équivalente à celle de l'air ($\simeq$ 21 % d'oxygène) ou dans le cas où ce mélange est en défaut d'oxygène, par rapport à l'air.

Au départ de propane, on obtiendra un mélange renfermant essentiellement du propylène et de l'acrylonitrile. L'acrylonitrile est un intermédiaire industriellement produit sur une large échelle, le propylène est une matière première traditionnellement utilisée pour produire de l'acrylonitrile et divers autres produits intermédiaires bien connus de l'homme de l'art.

Au départ d'isobutane, on obtiendra un mélange renfermant du méthacrylonitrile et de l'iso-butène ou des n-butènes.

Le procédé selon l'invention convient plus particulièrement à l'ammoxydation du propane.

Si l'hydrocarbure saturé mis en oeuvre peut être de qualité technique, il ne renfermera pas de quantités notables de composés à insaturation éthylénique. Ainsi le propane engagé ne renfermera de propylène qu'à l'état de traces.

Le procédé selon l'invention est réalisé sous forme de réaction en phase vapeur. En conséquence, n'importe quel dispositif convenant à la réalisation des réactions d'ammoxydation ou d'oxydation en phase vapeur peut être utilisé. Le procédé peut être conduit en continu ou en discontinu et il peut comprendre l'utilisation d'un lit fixe ou d'un lit fluidisé.

La température de réaction est en général comprise entre 300°C et 550°C et, de préférence entre 350°C et 500°C.

La pression totale du mélange réactionnel peut être supérieure ou égale à la pression atmosphérique. Elle est généralement comprise entre 1 et 6 bar et, de préférence entre 1 et 4 bar.

Le débit gazeux est fixé de telle sorte que la vitesse volumique horaire soit comprise entre 100 et 36000 h$^{-1}$ et, de préférence entre 200 et 20000 h$^{-1}$.

La vitesse volumique horaire se définit comme le rapport volume gazeux total/volume du catalyseur/heure.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Dans le procédé selon l'invention, les catalyseurs peuvent être préparés ou mis en oeuvre de la façon suivante.

On synthétise d'abord une phase active constituée d'un oxyde mixte renfermant les éléments V, Sb et Fe et/ou Ga et/ou In.

Cette phase active peut éventuellement être déposée sur un oxyde minéral ou mélangée avec ledit oxyde minéral, connu de l'homme de l'art, comme par exemple l'alumine, la silice, la silice-alumine, la zircone, la cérine, la magnésie, l'oxyde de titane, l'oxyde de niobium, en utilisant différentes techniques connues de l'homme de l'art, telles que l'imprégnation ou le dépôt par "slurry".

La phase catalytique, constituée de la phase active seule ou de la phase active déposée sur un oxyde minéral ou mélangée avec ledit oxyde minéral, peut ensuite être mise en oeuvre sous forme massique ou à l'état particulaire ; elle peut donc être utilisée sous forme de poudre ou être mise en forme, par exemple de billes, pastilles, extrudés, particules concassées, selon différentes techniques connues.

Pour une mise en oeuvre du procédé en lit fixe, ces techniques peuvent être, par exemple, le pastillage, l'enrobage sur un support inerte ou sur substrat céramique ou métallique de type monolithique.

Pour une mise en oeuvre du procédé en lit mobile ou en lit fluidisé, la phase catalytique est généralement mise en forme par atomisation, séchage et calcination.

La phase catalytique ainsi mise en forme ou se présentant sous forme de poudre constitue le catalyseur selon l'invention.

Lorsque la phase catalytique comprend la phase active déposée sur un oxyde minéral ou mélangée avec ledit oxyde minéral, une variante de préparation peut consister à réaliser en une seule étape, la synthèse de la phase active et son dépôt sur l'oxyde minéral ou son mélange avec ledit oxyde minéral.

Dans ce qui suit la synthèse de la phase active et son dépôt sur l'oxyde minéral ou son mélange avec ledit oxyde minéral seront d'abord décrits séparément, mais la description portera également sur la variante de synthèse de la phase active en présence de l'oxyde minéral.

La préparation des phases actives mises en oeuvre dans le procédé selon l'invention peut être réalisée par diverses techniques connues, telles que le mélange de sels ou d'oxydes convenables des différents éléments dans l'eau ou dans un autre solvant, suivi de l'évaporation jusqu'à siccité, ou de la précipitation par ajout d'une base telle que l'ammoniaque ou d'un acide tel que l'acide chlorhydrique, ou l'atomisation d'une suspension obtenue après mélange des sels convenables.

Les sels convenables les plus couramment employés contiennent des anions et des cations qui peuvent, par exemple, être décomposés par la chaleur lors des étapes ultérieures.

Comme exemples de sels ou oxydes convenables de vanadium, on peut citer le vanadate d'ammonium, les oxyhalogénures de vanadium tels que $VOCl_3$, $VOCl_2$, $(VO_2)Cl$, $VOCl$, $VOBr$, $VOBr_2$, $VOBr_3$, $VOF_3$, $VOF_2$, les halogénures de vanadium tels que $VF_3$, $VBr_3$, $VCl_2$, $VCl_3$, $VCl_4$, $VF_5$, $VF_4$, $VBr_2$, $VI_2$, le sulfate de vanadyle, l'acétylacétonate de vanadyle, l'acide méta-vanadique, l'hexacarbonyle de vanadium, le tri-isopropoxyde d'oxyde de vanadium, les oxydes de vanadium tels que $V_2O_5$, $V_7O_{13}$, $VO$, $VO_2$, $V_2O_3$, $V_3O_7$.

Comme exemples de sels ou oxydes convenables d'antimoine, on peut citer l'oxychlorure d'antimoine, les halogénures d'antimoine tels que $SbBr_3$, $SbCl_3$, $SbF_3$, $SbI_3$, $SbCl_5$, $SbF_5$, $SbI_5$, le sulfate d'antimoine, l'acétate d'antimoine, le tartrate d'antimoine, l'éthoxyde d'antimoine, le butoxyde d'antimoine, l'éthylèneglycoxyde d'antimoine, l'oxysulfate d'antimoine, les oxydes d'antimoine tels que $Sb_2O_3$, $Sb_2O_4$, $Sb_2O_5$.

Comme exemples de sels ou oxydes convenables de fer, on peut citer le nitrate de fer, le perchlorate de fer, l'oxychlorure de fer, les halogénures de fer tels que $FeCl_3$, $FeCl_2$, $FeBr_3$, $FeBr_2$, $FeF_3$, $FeF_2$, $FeI_2$, le phosphate de fer, le sulfate de fer, l'iodate de fer, le fer-pentacarbonyle, l'acétate de fer, l'acétylacétonate de fer, le citrate de fer, le formiate de fer, le gluconate de fer, le glycérophosphate de fer, le lactate de fer, le malate de fer, le méthoxyde de fer, l'oléate de fer, l'oxalate de fer, le tartrate de fer, le 2-éthyl-hexanoate de fer, les oxydes de fer tels que $Fe_2O_3$, $Fe_3O_4$, FeO.

Comme exemples de sels ou oxydes convenables de gallium, on peut citer le nitrate de gallium, le perchlorate de gallium, l'oxychlorure de gallium, les halogénures de gallium tels que $GaCl_3$, $GaCl_2$, $GaBr_3$, $GaF_3$, $GaI_3$, le sulfate de gallium, l'acétate de gallium, l'acétylacétonate de gallium, l'oxalate de gallium, les oxydes de gallium tels que $Ga_2O_3$, $Ga_2O$.

Comme exemples de sels ou oxydes convenables d'indium, on peut citer le nitrate d'indium, le perchlorate d'indium, les halogénures d'indium tels que $InCl_3$, $InCl_2$, $InCl$, $InBr_3$, $InBr_2$, $InBr$, $InF_3$, $InI_3$, $InI_2$, $InI$, le phosphate d'indium, le sulfate d'indium, l'iodate d'indium, l'acétate d'indium, l'acétylacétonate d'indium, le méthoxyde d'indium, les oxydes d'indium tels que $In_2O_3$, $In_2O$, InO.

La synthèse de la phase active est généralement réalisée par la méthode dite d'évaporation de la façon suivante : on prépare une suspension aqueuse des sels ou oxydes convenables, on évapore en chauffant entre 20 et 100°C jusqu'à l'obtention d'une pâte visqueuse que l'on sèche. Le précurseur ainsi obtenu peut ensuite être broyé et calciné entre 200 et 1000°C. La phase active ainsi obtenue après refroidissement peut ensuite être broyée pour que sa granulométrie n'excède pas 400 µm environ.

Le précurseur peut aussi être obtenu selon une variante comprenant la précipitation avec addition, par exemple, d'ammoniaque ou d'acide chlorhydrique, au cours ou en fin de mélange des sels ou oxydes. Il est préférable de chauffer la suspension entre 20 et 100°C pour parfaire la précipitation des espèces.

La suspension obtenue peut être évaporée selon les conditions décrites ci-avant, ou filtrée et lavée. La pâte ou le gâteau de filtration, obtenus respectivement par évaporation ou par filtration, sont ensuite séchés, broyés et calcinés selon les conditions décrites ci-avant dans le cadre de la méthode d'évaporation, pour donner la phase active.

Cette phase active peut éventuellement être déposée sur un ou plusieurs oxydes minéraux ou être mélangée avec le ou lesdits oxydes minéraux, connus de l'homme de l'art. A titre d'exemples d'oxydes minéraux susceptibles de convenir à la préparation de catalyseurs utilisables dans le cadre du procédé selon l'invention, on peut citer de manière non limitative l'alumine, la silice, la silice-alumine, la zircone, la cérine, la magnésie, l'oxyde de titane, l'oxyde de niobium.

Le dépôt sur ces oxydes ou le mélange avec ces oxydes minéraux peuvent s'effectuer par différentes techniques connues, comme par exemple l'imprégnation ou le dépôt par "slurry".

La quantité de phase active, qui peut varier dans de larges limites, est en pratique comprise entre 5 et 100 % et de préférence entre 10 et 50 % en poids par rapport à l'ensemble phase active + oxyde minéral.

Une autre méthode couramment utilisée dans le cadre de l'invention est le mélange de l'oxyde minéral avec les sels ou oxydes convenables des différents éléments de la phase active selon les conditions décrites ci-avant dans le cadre de la préparation des phases actives. Une fois ce mélange réalisé, le précurseur peut être obtenu par les méthodes dites d'évaporation ou de précipitation, puis la pâte ou le gâteau de filtration obtenus sont séchés, broyés et calcinés selon les conditions décrites ci-avant dans le cadre de la préparation des phases actives.

La phase active seule ou déposée sur un oxyde minéral ou mélangée avec un oxyde minéral tels que décrits précédemmment, constituent la phase catalytique.

Les phases catalytiques en cause peuvent être mises en oeuvre sous forme massique ou à l'état particulaire. Ces phases peuvent donc être utilisées sous forme de poudre ou être mises, par exemple, sous forme de billes, pastilles, extrudés, particules concassées, selon différentes techniques connues.

Pour une mise en oeuvre du procédé en lit fixe, on peut citer à titre d'exemples de techniques pouvant convenir à la préparation des catalyseurs utilisables dans le cadre du procédé selon l'invention : le pastillage, l'enrobage sur un support inerte ou sur un substrat céramique ou métallique de type monolithique.

Les phases catalytiques selon l'invention peuvent par exemple être mises en forme par compression, de façon à obtenir des pastilles. Ces pastilles peuvent ensuite être éventuellement concassées en éclats. Les valeurs précises de la pression, du diamètre et de l'épaisseur des pastilles, et de la granulométrie des éclats pourront être choisies par l'homme de l'art en fonction de la perte de charge admissible dans le réacteur.

Les phases catalytiques selon l'invention peuvent également être déposées sur un support inerte ou l'enrober. La nature de ce support n'est pas critique dès lors qu'il est chimiquement inerte vis-à-vis des réactifs dans les conditions réactionnelles choisies. A titre d'exemples de supports susceptibles de convenir à la préparation de catalyseurs utilisables dans le cadre du procédé selon l'invention, on peut citer : la silice, l'alumine, la silice-alumine, l'argile frittée, le carborandum, la magnésie, le silicate de magnésium, la terre de diatomée. Ce support est de préférence non poreux et peut notamment être à base d'oxyde réfractaire sous forme particulaire, le support le plus couramment employé étant à base d'argile. Ce support peut par exemple être constitué de billes d'argile, inertes, pleines, solides et rugueuses, de diamètre compris entre 0.5 et 6 mm. La valeur précise du diamètre des billes pourra être choisie en fonction de la perte de charge admissible dans le réacteur. Ce support peut également être rendu non poreux par émaillage.

Ce support peut également être un substrat céramique, ledit substrat étant de préférence sous forme d'une structure inerte et rigide de type monolithique comprenant des canaux ou conduits. De tels supports sont bien connus et ont été largement décrits dans la littérature. Les substrats en matières céramiques utilisés sont notamment ceux comprenant comme matière principale la cordiérite, l'alumine, la mullite, la porcelaine, les carbures de bore ou de silicium.

Ce support peut également être un substrat métallique. De tels supports sont bien connus. Les substrats métalliques convenables sont notamment ceux obtenus à partir d'alliages de fer, de nickel et de chrome, ou ceux obtenus à partir d'alliages de fer, de chrome, aluminium et cobalt tels que ceux connus sous la marque KANTHAL ou ceux obtenus à partir d'alliages de fer, de chrome, d'aluminium et d'yttrium connus sous la marque FECRALLOY. Le métal peut aussi être de l'acier carboné ou de la fonte simple.

Lorsqu'on fait appel à un catalyseur enrobé, la quantité de phase catalytique qui peut varier dans de larges limites, est en pratique comprise entre 1 et 50 % et de préférence entre 3 et 35 % en poids par rapport à l'ensemble support + phase catalytique.

Ainsi, certains catalyseurs, utiles pour une mise en oeuvre du procédé en lit fixe, peuvent être obtenus par enrobage de manière connue en soi, des phases catalytiques, intermédiaires ou finies, broyées. Cette méthode classique consiste à déposer autour de billes inertes, mais rugueuses, une couche de phase catalytique intermédiaire ou finie. Une fois les billes recouvertes de la quantité voulue de la phase catalytique, elles sont séchées par de l'air chaud, entre 70 et 150°C pendant au moins 30 minutes, puis introduites dans un four pour être calcinées entre 300 et 600°C de préférence entre 450 et 500°C, pendant au moins 3 heures.

Certains catalyseurs utiles pour une mise en oeuvre du procédé selon l'invention en lit mobile ou en lit fluidisé peuvent être obtenus par la technique connue en soi du séchage par atomisation en atmosphère, de préférence non réductrice. Par une telle opération, le cas échéant suivie d'une calcination à une température de l'ordre de 400 à 1100°C, on obtient des poudres de forme sphérique de diamètre compris entre 5 et 700 $\mu$m. Des poudres constituées pour au moins 80 % en poids de particules dont la dimension est comprise entre 5 et 200 $\mu$m sont préférées dans le cadre d'une utilisation en lit fluidisé.

La phase catalytique ainsi mise en oeuvre sous forme massique ou à l'état particulaire, constitue le catalyseur selon l'invention.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti, du propylène, des hydrocarbures légers et le cas échéant du $CO_2$. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

## EXEMPLE 1 - Préparation du catalyseur ($A_1$) selon l'invention, de formule empirique (II) suivante : V $Sb_{3,5}Fe_2Ox/Al_2O_3$(25/75 % en poids) (II)

a) On prépare une solution (a) de vanadate d'ammonium par dissolution de 2,34 g de $NH_4VO_3$ dans 400 cm$^3$ d'eau permutée, une solution (b) de nitrate de fer par dissolution de 16,2 g de Fe $(NO_3)_3$, 9 $H_2O$ dans 70 cm$^3$ d'eau permutée et une suspension (c) de chlorure d'antimoine par dissolution de 10,2 g de $Sb_2O_3$ dans 250 cm$^3$ d'acide chlorhydrique 1 N. On ajoute sous agitation, à 70°C environ, la solution (b) à la solution (a), puis on ajoute la suspension (c). On ajoute ensuite, à 90°C environ, 47,3 g de $Al_2O_3$. On maintient à 90°C pendant 6 h, on évapore à sec, on sèche à 120°C pendant 15 h environ, puis on calcine à 550°C pendant 10 h.

b) Le produit de formule (II) ainsi obtenu est ensuite comprimé sous une pression de 4300 kg/cm$^2$. On obtient ainsi des pastilles de 3 cm de diamètre et d'environ 0,5 cm d'épaisseur. Ces pastilles sont concassées en éclats de granulométrie comprise entre 0,3 et 0,8 cm constituant le catalyseur $A_1$ conforme à l'invention.

## EXEMPLE 2 - Préparation du catalyseur ($A_2$) selon l'invention, de formule empirique suivante : V $Sb_{3,5}Fe_2Ox/Al_2O_3$(25/75 % en poids) enrobé sur argile

Le produit de formule (II) obtenu dans l'étape a) de l'exemple 1 est ensuite utilisé de la manière suivante.

On saupoudre lentement 15 g dudit produit préparé précédemment sur 100 g de support inerte composé de billes d'argile de diamètre moyen 4,8 mm, préalablement placées dans un drageoir en rotation et humidifiées par du glucose en solution aqueuse à 10 % ; dès que les billes sont sèches à l'extérieur, on pulvérise une petite quantité de la solution de glucose. Puis on saupoudre à nouveau le produit de formule (II) sur les billes. Ces opérations sont poursuivies alternativement jusqu'à enrobage de la totalité du produit de formule (II).

On sèche ensuite à 120°C pendant 2 heures et on effectue une calcination de 6 heures à 480°C.

Le catalyseur $A_2$ ainsi obtenu est constitué de 10,4 % en poids de V $Sb_{3,5}$ $Fe_2$ Ox/$Al_2O_3$ (25/75 % en poids) enrobé sur billes d'argile.

**EXEMPLE 3 - Préparation du catalyseur ($B_1$) selon l'invention, de formule empirique (III) suivante :**
**V $Sb_{3,5}Ga_2Ox/Al_2O_3$(25/75 % en poids)**

a) On prépare le produit de formule (III) V $Sb_{3,5}$ $Ga_2$ Ox/$Al_2O_3$ de la manière suivante : on prépare une solution a) de vanadate d'ammonium par dissolution de 2,34 g de $NH_4$ $VO_3$ dans 400 $cm^3$ d'eau permutée, une solution b) de nitrate de gallium par dissolution de 10,2 g de Ga($NO_3$)$_3$ dans 50 $cm^3$ d'eau permutée et une suspension c) de chlorure d'antimoine par dissolution de 10,2 g de $Sb_2O_3$ dans 250 $cm^3$ d'acide chlorhydrique 1 N. On ajoute sous agitation, à 80°C environ, la solution b) à la solution a), puis on ajoute la suspension c). On ajoute ensuite, à 90°C environ, 47,3 g de $Al_2$ $O_3$. On chauffe à 105°C pendant 6 heures, on évapore à sec, on sèche à 120°C pendant 15 heures environ, puis on calcine à 550°C pendant 10 heures.

b) Le produit de formule III ainsi préparé en a) est ensuite comprimé sous une pression de 4300 kg/$cm^2$. On obtient ainsi des pastilles de 3 cm de diamètre et d'environ 0,5 cm d'épaisseur. Ces pastilles sont concassées en éclats de granulométrie comprise entre 0,3 cm et 0,8 cm constituant le catalyseur $B_1$ conforme à l'invention.

**EXEMPLE 4 - Préparation du catalyseur ($B_2$) selon l'invention, de formule empirique :**
**V $Sb_{3,5}Ga_2Ox/Al_2O_3$(25/75 % en poids) enrobé sur billes d'argile**

Le produit de formule (III) obtenu dans l'étape a) de l'exemple 3 est utilisé de la manière suivante.

15 g du produit de formule (III) ainsi préparé sont saupoudrés lentement sur 100 g de support inerte, composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit de formule (III) sur les billes. On poursuit ces opérations alternativement jusqu'à enrobage de la totalité dudit produit. On sèche ensuite à 120°C pendant 2 h et on calcine à 480°C pendant 6 h.

Le catalyseur ($B_2$) ainsi obtenu, conforme à l'invention, est constitué de 4,3 % en poids de V $Sb_{3,5}$ $Ga_2$ Ox/$Al_2O_3$ (25/75 % en poids) enrobé sur billes d'argile.

**EXEMPLE 5 - Préparation du catalyseur (C) selon l'invention, de formule empirique (IV) suivante :**
**V $Sb_{3,5}In_2Ox/Al_2O_3$(25/75 % en poids) (IV)**

a) On prépare une solution (a) de vanadate d'ammonium par dissolution de 1,5 g de $NH_4VO_3$ dans 250 $cm^3$ d'eau permutée, une suspension (b) de chlorure d'antimoine par dissolution de 6,53 g de $Sb_2O_3$ dans 13,4 $cm^3$ d'acide chlorhydrique à 37 % et 150 $cm^3$ d'eau permutée, et une solution (c) de nitrate d'indium par dissolution de 10 g de In ($NO_3$)$_3$, 5 $H_2O$ dans 50 $cm^3$ d'eau permutée. On ajoute sous agitation, à 80°C environ, la solution (c) à la solution (a), puis la suspension (b), puis 30,3 g de $Al_2O_3$. On maintient à cette température pendant 6 h, on évapore à sec, on sèche à 120°C pendant 15 h environ, puis on calcine à 550°C pendant 10 h.

b) Le produit de formule (IV) ainsi obtenu est ensuite comprimé sous une pression de 4300 kg/$cm^2$. On obtient ainsi des pastilles de 3 cm de diamètre et d'environ 0,5 cm d'épaisseur. Ces pastilles sont concassées en éclats de granulométrie comprise entre 0,3 et 0,8 cm constituant le catalyseur C conforme à l'invention.

**EXEMPLE 6 - Préparation du catalyseur (D) selon l'invention, de formule empirique (V) suivante :**
**V $Sb_5Fe_2Ox/Al_2O_3$(25/75 % en poids) (V)**

a) On prépare une solution (a) de vanadate d'ammonium par dissolution de 2,34 g de $NH_4VO_3$ dans 400 $cm^3$ d'eau permutée, une solution (b) de nitrate de fer par dissolution de 16,2 g de Fe ($NO_3$)$_3$, 9 $H_2O$ dans 70 $cm^3$ d'eau permutée et une suspension (c) de chlorure d'antimoine par dissolution de 14,6 g de $Sb_2O_3$ dans 30 $cm^3$ d'acide chlorhydrique à 37 % et 300 $cm^3$ d'eau permutée. On ajoute sous agitation, à 70°C environ, la solution (b) à la solution (a), puis on ajoute la suspension (c). On porte à reflux et on ajoute 47,3 g de $Al_2O_3$. On maintient à cette température pendant 6 h, on évapore à sec, on sèche à 120°C pendant 15 h environ, puis on calcine à 550°C pendant 10 h.

b) Le produit de formule (V) ainsi obtenu est ensuite comprimé sous une pression de 4300 kg/$cm^2$. On obtient ainsi des pastilles de 3 cm de diamètre et d'environ 0,5 cm d'épaisseur. Ces pastilles sont concassées en éclats de granulométrie comprise entre 0,3 et 0,8 cm constituant le catalyseur D conforme à l'invention.

**ESSAI COMPARATIF 1- Préparation du catalyseur (E) non conforme à l'invention, de formule empirique suivante** :

**V Sb$_{3,5}$Fe$_{0,1}$Ox/Al$_2$O$_3$(25/75 % en poids)**

On prépare un produit de composition V Sb$_{3,5}$ Fe$_{0,1}$ Ox/Al$_2$ O$_3$ (25/75 % en poids) selon le protocole opératoire suivant. On prépare une solution (a) de vanadate d'ammonium par dissolution de 2,34 g de NH$_4$VO$_3$ dans 400 cm$^3$ d'eau permutée, une solution b) de nitrate de fer par dissolution de 0,81 g de Fe (NO$_3$)$_3$, 9 H$_2$O dans 70 cm$^3$ d'eau permutée et une suspension (c) de chlorure d'antimoine par dissolution de 10,2 g de Sb$_2$O$_3$ dans 21 cm$^3$ d'acide chlorhydrique à 37 % et 250 cm$^3$ permutée. On ajoute sous agitation à 70°C environ, la solution (b) à la solution (a), puis on ajoute la suspension c). On porte à reflux et on ajoute 47,3 g de Al$_2$O$_3$. On maintient à cette température pendant 6 h, on évapore à sec, on séche à 120°C pendant 15 h environ, puis on calcine à 550°C pendant 10 h.

Ce produit est ensuite comprimé sous une pression de 4300 kg/cm$^2$. On obtient ainsi des pastilles de 3 cm de diamètre et environ 0,5 cm d'épaisseur. Ces pastilles sont ensuite concassées en éclats de granulométrie comprise entre 0,3 et 0,8 cm, constituant le catalyseur (E), de composition V Sb$_{3,5}$ Fe$_{0,1}$ Ox/Al$_2$ O$_3$ (25/75 % en poids), non conforme à l'invention.

**ESSAI COMPARATIF 2- Préparation du catalyseur (F) non conforme à l'invention, de formule empirique suivante** :

**V Sb$_{3,5}$Ox/Al$_2$O$_3$(25/75 % en poids)**

On prépare un produit de composition V Sb$_{3,5}$ Ox/Al$_2$ O$_3$ (25/75 % en poids) selon le protocole opératoire suivant. On prépare une solution (a) de vanadate d'ammonium par dissolution de 2,34 g de NH$_4$VO$_3$ dans 400 cm$^3$ d'eau permutée et une suspension (b) de chlorure d'antimoine par dissolution de 10,2 g de Sb$_2$O$_3$ dans 21 cm$^3$ d'acide chlorhydrique à 37 % et 230 cm$^3$ d'eau permutée. On ajoute sous agitation à 80-90°C environ, la suspension (b) à la solution (a), puis 47,3 g de Al$_2$O$_3$. On maintient à cette température pendant 6 h, on évapore à sec, on séche à 120°C pendant 15 h environ, puis on calcine à 550°C pendant 10 h.

Ce produit est ensuite comprimé sous une pression de 4300 kg/cm$^2$. On obtient ainsi des pastilles de 3 cm de diamètre et environ 0,5 cm d'épaisseur. Ces pastilles sont ensuite concassées en éclats de granulométrie comprise entre 0,3 et 0,8 cm, constituant le catalyseur (F), de composition V Sb$_{3,5}$ Ox/Al$_2$ O$_3$ (25/75 % en poids), non conforme à l'invention.

**MODE OPERATOIRE GENERAL DES TESTS D'AMMOXYDATION**

L'échantillon de catalyseur est préalablement porté sur un banc de mesure à la température de 150°C sous balayage d'hélium pendant 10 min, puis il est soumis à un flux gazeux dont la composition sera précisée pour chaque exemple et qui renferme du propane, de l'ammoniac, de l'oxygène, de la vapeur d'eau et de l'hélium.

La pression totale du mélange réactionnel comprise entre 1 et 6 bar, sera également précisée pour chaque exemple.

Le débit total gazeux est défini de façon à avoir une vitesse volumique horaire (VVH) comprise entre 100 et 36000 h$^{-1}$, dont la valeur précise sera indiquée pour chaque exemple.

Volume de catalyseur : (phase catalytique + support éventuel) : 25 cm$^3$.

Lorsque le volume du catalyseur mis en oeuvre sera différent de cette valeur, la valeur particulière sera indiquée dans les exemples.

Le principe du test d'ammoxydation du propane est le suivant :

- On porte le catalyseur à une température T$_1$, par exemple 300°C, et après 30 min de stabilisation à la température T$_1$, on détermine par chromatographie en phase gazeuse la composition du mélange en sortie de réacteur.
- On calcule les pourcentages de conversion et les sélectivités obtenus sur le catalyseur examiné à la température d'entrée T$_1$ par des relations du type :

conversion du propane (en moles %) = propane transformé/

propane introduit

sélectivité en acrylonitrile (en moles %) = propane

transformé en acrylonitrile/propane converti

- On porte ensuite le catalyseur de 300 à 550°C par incrément de 20°C et on détermine toutes les 40 min les pourcentages de conversion et les sélectivités.

Dans les exemples ci-après, les conventions suivantes sont utilisées :

$TTC_3H8 =$           conversion du propane
$SACN =$           sélectivité en acrylonitrile
$SACN+C_3H_6 =$           sélectivité en acrylonitrile et propylène
$SCOX =$           sélectivité en monoxyde et dioxyde de carbone
$SAMMOX =$           sélectivité en acétonitrile, en acide cyanhydrique et autres sous-produits d'(amm)oxydation
$SC_1C_2 =$           sélectivité en méthane, éthane et éthylène.

### EXEMPLE 7 ET ESSAI COMPARATIF 3

Mesure des performances des catalyseurs ($A_1$) et(E).
Les conditions opératoires mises en oeuvre sont les suivantes

- Vitesse volumique horaire = 1000h$^{-1}$
- Pression totale = 1,3 bar
- Composition volumique du mélange réactionnel :

$C_3H_8 = 48$ %
$NH_3 = 9$ %
$O_2 = 18$ %
$H_2O = 20$ %
$He = 5$ %

Les conditions de température et les résultats obtenus sont rassemblés dans le tableau 1 ci-après.

| Essais | Exemple 7 | | | | Essai comparatif 3 | | | |
|---|---|---|---|---|---|---|---|---|
| Catalyseur utilisé | $A_1$ | | | | E | | | |
| Température (en °C) | 380 | 400 | 420 | 440 | 380 | 400 | 410 | 430 |
| $TTC_3H_8$ % | 9,5 | 12 | 11 | 10 | 7 | 12 | 13 | 19 |
| SACN % | 34 | 36 | 38 | 33 | 2 | 10 | 9 | 17 |
| SACN + $C_3H_6$ % | 60 | 63 | 71 | 76 | 49 | 42 | 41 | 41 |
| SAMMOX % | 39 | 36 | 26 | 19 | 22 | 44 | 44 | 54 |
| $SC_1C_2$ % | 0 | 0 | 0 | 1 | 0 | 0 | 0 | 0 |
| SCOX % | 0 | 0 | 2 | 4 | 29 | 14 | 15 | 5 |
| **TABLEAU 1** | | | | | | | | |

### EXEMPLE 8 ET ESSAI COMPARATIF 4

Mesure des performances des catalyseurs ($A_1$) et (E).
Les conditions opératoires mises en oeuvre sont les suivantes :

- Vitesse volumique horaire = 1000h$^{-1}$
- Pression totale = 1,3 bar

- Composition volumique du mélange réactionnel :

$C_3H_8 = 7,5\ \%$
$NH_3 = 15\ \%$
$O_2 = 15\ \%$
$H_2O = 20\ \%$
$He = 42,5\ \%$

Les conditions de température et les résultats obtenus sont rassemblés dans le tableau 2 ci-après.

**TABLEAU 2**

| Essais | Exemple 8 | | | | | Essai comparatif 4 | | | | |
|---|---|---|---|---|---|---|---|---|---|---|
| Catalyseur utilisé | $A_1$ | | | | | E | | | | |
| Température (en °C) | 380 | 400 | 420 | 440 | 460 | 380 | 400 | 420 | 440 | 460 |
| TTC3H8 % | 2 | 5 | 15 | 31 | 38 | 4 | 8 | 15 | 26 | 42 |
| SACN % | 18 | 34 | 66 | 74 | 66 | 9 | 18 | 31 | 27 | 34 |
| SACN + C3H6 % | 79 | 65 | 78 | 81 | 75 | 44 | 44 | 50 | 39 | 43 |
| SAMMOX % | 17 | 33 | 18 | 18 | 10 | 56 | 50 | 41 | 38 | 40 |
| SC1C2 % | 0 | 0 | 4 | 0 | 0 | 0 | 0 | 0 | 0 | 0 |
| SCOX % | 4 | 2 | 0 | 0 | 15 | 0 | 7 | 10 | 22 | 17 |

**EXEMPLES 9 ET 10**

Mesure des performances du catalyseur $(B_2)$.
Les conditions opératoires mises en oeuvre sont les suivantes :

- Vitesse volumique horaire = 1000h$^{-1}$

10

- Pression totale = 1,3 bar

La composition volumique du mélange réactionnel, les conditions de température (T°C) et les résultats obtenus sont rassemblés dans le tableau 3 ci-après.

| Exemples | $C_3H_8$ % | $NH_3$ % | $O_2$ % | $H_2O$ % | He % | T°C | TT $C_3H_8$ % | SACN % | SACN + $C_3H_6$ % | SAMMOX % | $SC_1C_2$ % | SCOX % |
|---|---|---|---|---|---|---|---|---|---|---|---|---|
| Exemple 9 | 7,5 | 15 | 15 | 20 | 42,5 | 500 | 12,5 | 10 | 61 | 28 | 9 | 1 |
| Exemple 10 | 25 | 10 | 25 | 20 | 20 | 480 | 6 | 10 | 68 | 27 | 4 | 0 |

TABLEAU 3

## EXEMPLE 11

Mesure des performances du catalyseur ($A_2$).
Les conditions opératoires mises en oeuvre sont les suivantes :

- Vitesse volumique horaire = $1000h^{-1}$
- Pression totale = 1,3 bar
- Composition volumique du mélange réactionnel :

  $C_3H_8$ = 48 %
  $NH_3$ = 9 %
  $O_2$ = 18 %
  $H_2O$ = 20 %
  He = 5 %

Les conditions de température et les résultats obtenus sont rassemblés dans le tableau 4 ci-après.

| | 440°C | 460°C | 480°C |
|---|---|---|---|
| $TTC_3H_8$ % | 2 | 5 | 8,5 |
| SACN % | 15 | 38 | 46 |
| SACN + $C_3H_6$ % | 86 | 86 | 82 |
| SAMMOX % | 6 | 11 | 15 |
| $SC_1C_2$ % | 8 | 3 | 3 |
| SCOX % | 0 | 0 | 0 |
| **TABLEAU 4** | | | |

## EXEMPLES 12 ET 13

Mesure des performances des catalyseurs ($B_1$) et (C).
Les conditions opératoires mises en oeuvre sont les suivantes :

- Vitesse volumique horaire = $1000h^{-1}$
- Pression totale = 1,3 bar
- Composition volumique du mélange réactionnel :

  $C_3H_8$ = 7,5 %
  $NH_3$ = 15 %
  $O_2$ = 15 %
  $H_2O$ = 20 %
  He = 42,5 %

Les conditions de température et les résultats obtenus sont rassemblés dans le tableau 5 ci-après.

| Exemples | Exemple 12 | | | | Exemple 13 | | | |
|---|---|---|---|---|---|---|---|---|
| Catalyseur utilisé | $B_1$ | | | | C | | | |
| Température (en °C) | 400 | 420 | 440 | 460 | 380 | 400 | 420 | 440 |
| $TTC_3H_8$ % | 14 | 29 | 43 | 49 | 5 | 11 | 23 | 32 |
| SACN % | 21 | 36 | 48 | 56 | 12 | 31 | 56 | 56 |
| SACN + $C_3H_6$ % | 39 | 46 | 57 | 66 | 61 | 57 | 71 | 69 |
| SAMMOX % | 58 | 43 | 37 | 26 | 39 | 37 | 25 | 17 |
| $SC_1C_2$ % | 0 | 0 | 0 | 0 | 0 | 0 | 4 | 1 |
| SCOX % | 3 | 11 | 7 | 8 | 0 | 5 | 0 | 14 |
| **TABLEAU 5** | | | | | | | | |

## EXEMPLE 14 ET ESSAI COMPARATIF 5

Mesure des performances des catalyseurs (C) et (F).
Les conditions opératoires mises en oeuvre sont les suivantes :

- Vitesse volumique horaire = 1000h$^{-1}$
- Pression totale = 1,3 bar
- Composition volumique du mélange réactionnel :

$C_3H_8$ = 40 %
$NH_3$ = 15 %
$O_2$ = 15 %
$H_2O$ = 20 %
He = 10 %

Les conditions de température et les résultats obtenus sont rassemblés dans le tableau 6 ci-après.

| Essais | Exemple 14 | | | | Essai comparatif 5 | | | |
|---|---|---|---|---|---|---|---|---|
| Catalyseur utilisé | C | | | | F | | | |
| Température (en °C) | 400 | 420 | 440 | 460 | 400 | 420 | 440 | 460 |
| $TTC_3H_8$ % | 7 | 11 | 11 | 12 | 15 | 15 | 15 | 17 |
| SACN % | 33 | 35 | 39 | 36 | 28 | 21 | 16 | 14 |
| SACN + $C_3H_6$ % | 74 | 68 | 71 | 70 | 68 | 64 | 63 | 62 |
| SAMMOX % | 21 | 23 | 20 | 19 | 26 | 28 | 25 | 23 |
| $SC_1C_2$ % | 0,5 | 1 | 2 | 4 | 0,5 | 0 | 1 | 5 |
| SCOX % | 4 | 8 | 7 | 7 | 6 | 8 | 11 | 10 |
| **TABLEAU 6** | | | | | | | | |

## EXEMPLE 15

Mesure des performances du catalyseur (C).
Les conditions opératoires mises en oeuvre sont les suivantes :

- Vitesse volumique horaire = 1000h$^{-1}$
- Pression totale = 1,3 bar
- Composition volumique du mélange réactionnel :

$C_3H_8$ = 25 %
$NH_3$ = 25 %
$O_2$ = 10 %
$H_2O$ = 20 %
He = 20 %

Les conditions de température et les résultats obtenus sont rassemblés dans le tableau 7 ci-après.

| Température | 400°C | 420°C | 440°C | 460°C | 480°C |
|---|---|---|---|---|---|
| $TTC_3H_8$ % | 7 | 13 | 13 | 13 | 13 |
| SACN % | 34 | 32 | 37 | 42 | 46 |
| SACN + $C_3H_6$ % | 70 | 59 | 63 | 68 | 74 |
| SAMMOX % | 30 | 40 | 30 | 26 | 19 |
| $SC_1C_2$ % | 0 | 0 | 7 | 6 | 7 |
| SCOX % | 0 | 0 | 0 | 0 | 0 |
| **TABLEAU 7** | | | | | |

**EXEMPLE 16**

Mesure des performances du catalyseur ($A_1$).
Les conditions opératoires mises en oeuvre sont les suivantes :

- Vitesse volumique horaire = 1000h$^{-1}$
- Pression totale = 1,3 bar
- Volume du catalyseur : 19 cm$^3$
- Composition volumique du mélange réactionnel :

$C_3H_8$ = 25 %
$NH_3$ = 25 %
$O_2$ = 10 %
$H_2O$ = 20 %
He = 20 %

Les conditions de température et les résultats obtenus sont rassemblés dans le tableau 8 ci-après.

| Température | 380°C | 400°C | 420°C | 440°C | 460°C |
|---|---|---|---|---|---|
| TT$C_3H_8$ % | 3 | 7 | 10 | 10 | 11 |
| SACN % | 30 | 51 | 52 | 45 | 44 |
| SACN + $C_3H_6$ % | 78 | 82 | 85 | 85 | 85 |
| SAMMOX % | 23 | 19 | 15 | 15 | 15 |
| S$C_1C_2$ % | 0 | 0 | 0 | 0 | 0 |
| SCOX % | 0 | 0 | 0 | 0 | 0 |
| **TABLEAU 8** | | | | | |

**EXEMPLE 17**

Mesure des performances du catalyseur (D).
Les conditions opératoires mises en oeuvre sont les suivantes :

- Vitesse volumique horaire = 1000h$^{-1}$
- Pression totale = 1,3 bar
- Composition volumique du mélange réactionnel :

$C_3H_8$ = 7,5 %
$NH_3$ 15 %
$O_2$ = 15 %
$H_2O$ = 20 %
He = 42,5 %

Les conditions de température et les résultats obtenus sont rassemblés dans le tableau 9 ci-après.

| Température (en °C) | 360 | 380 | 400 | 420 | 440 | 460 |
|---|---|---|---|---|---|---|
| TT$C_3H_8$ % | 3 | 9 | 12 | 23 | 34 | 45 |
| SACN % | 16 | 41 | 60 | 72 | 76 | 76 |
| SACN + $C_3H_6$ % | 32 | 48 | 68 | 77 | 79 | 80 |
| SAMMOX % | 68 | 53 | 31 | 19 | 13 | 12 |
| S$C_1C_2$ % | 0 | 0 | 0 | 0 | 0 | 0 |
| SCOX % | 0 | 0 | 1 | 5 | 8 | 8 |
| **TABLEAU 9** | | | | | | |

## EXEMPLE 18

Mesure des performances du catalyseur (D).
Les conditions opératoires mises en oeuvre sont les suivantes :

- Vitesse volumique horaire = 1000h$^{-1}$
- Pression totale = 1,3 bar
- Composition volumique du mélange réactionnel :

$C_3H_8$ = 20 %
$NH_3$ = 20 %
$O_2$ = 20 %
$H_2O$ = 20 %
$He$ = 20 %

Les conditions de température et les résultats obtenus sont rassemblés dans le tableau 10 ci-après.

| Température | 370°C | 380°C | 390°C | 400°C |
|---|---|---|---|---|
| $TTC_3H_8$ % | 2 | 4 | 7 | 11 |
| SACN % | 18 | 28 | 43 | 56 |
| SACN + $C_3H_6$ % | 42 | 49 | 58 | 68 |
| SAMMOX % | 58 | 51 | 42 | 32 |
| $SC_1C_2$ % | 0 | 0 | 0 | 0 |
| SCOX % | 0 | 0 | 0 | 1 |
| **TABLEAU 10** | | | | |

## Revendications

1. Procédé d'ammoxydation d'alcanes en phase vapeur en présence d'un catalyseur solide comportant au moins une phase active, caractérisé en ce que la dite phase active répond à la formule empirique suivante (I) :

$$VSb_aM_bOx \qquad\qquad (I)$$

dans laquelle :

- a représente un nombre entier ou fractionnaire égal ou supérieur à 1,
- M représente un atome de fer et/ou de gallium et/ou d'indium,
- b représente un nombre entier ou fractionnaire égal ou supérieur à 0,5,
- x représente un nombre entier ou fractionnaire déterminé par le degré d'oxydation des autres éléments de la phase active.

2. Procédé selon la revendication 1, caractérisé en ce que ladite phase active répond à la formule (I) dans laquelle :

- le symbole a représente un nombre entier ou fractionnaire jusqu'à 20 et est de préférence compris entre 1 et 10,
- le symbole b représente un nombre entier ou fractionnaire jusqu'à 20 et est de préférence compris entre 1 et 10.

3. Procédé selon l'une des revendications 1 ou 2, caractérisé en ce que l'alcane est le propane.

4. Procédé selon l'une des revendications 1 à 3, caractérisé en ce que la réaction est conduite en présence de vapeur d'eau.

5. Procédé selon l'une des revendications 1 à 4, caractérisé en ce que la température de réaction est comprise entre 300°C et 550°C et de préférence entre 350°C et 500°C.

**6.** Procédé selon l'une des revendications 1 à 5, caractérisé en ce que la pression totale est comprise entre 1 et 6 bar.

**7.** Procédé selon l'une des revendications 1 à 6, caractérisé en ce que la vitesse volumique horaire est comprise entre 100 et 36 000 h$^{-1}$.

**8.** Procédé selon l'une des revendications 1 à 7, caractérisé en ce que dans le gaz réactif (mélange d'hydrocarbure saturé, d'ammoniac et d'oxygène) la teneur en hydrocarbure saturé est comprise entre 5 et 70 %, la teneur en ammoniac est comprise entre 3 et 50 % et la teneur en oxygène est comprise entre 3 et 45 %.

**9.** Procédé selon l'une des revendications 1 à 8, caractérisé en ce que la composition du mélange gazeux est en dehors du domaine d'explosivité.

**10.** Procédé selon l'une des revendications 1 à 9, caractérisé en ce que le catalyseur solide renferme, outre ladite phase active, au moins un oxyde minéral.

**11.** Procédé selon la revendication 10, caractérisé en ce que l'oxyde minéral est choisi parmi l'alumine, la silice, la silice-alumine, la zircone, la cérine, la magnésie, l'oxyde de titane, l'oxyde de niobium et leurs mélanges.

**12.** Procédé selon l'une des revendications 1 à 9, caractérisé en ce que le catalyseur solide renferme, outre ladite phase active, un support inerte ou un substrat céramique ou métallique de type monolithique.

**13.** Procédé selon l'une des revendications 10 ou 11, caractérisé en ce que le catalyseur solide renferme également un support inerte ou un substrat céramique ou métallique de type monolithique.

**14.** Procédé selon l'une des revendications 10 ou 11, caractérisé en ce que la phase active du catalyseur représente de 5 % à 100 % en poids du poids dudit catalyseur et de préférence de 10 % à 50 % en poids par poids.

**15.** Procédé selon l'une des revendications 12 ou 13, caractérisé en ce que la phase active ou la phase catalytique représente de 1 % à 50 % en poids du poids du catalyseur et de préférence de 3 % à 35 % en poids par poids.

**Claims**

**1.** Process for the ammoxidation of alkanes in the vapour phase in the presence of a solid catalyst comprising at least one active phase, characterised in that the said active phase corresponds to the following empirical formula (I):

$$VSb_aM_bO_x \qquad\qquad (I)$$

in which:

-   a represents a whole or fractional number equal to or greater than 1,
-   M represents an iron and/or gallium and/or indium atom,
-   b represents a whole or fractional number equal to or greater than 0.5,
-   x represents a whole or fractional number specified by the oxidation number of the other elements of the active phase.

**2.** Process according to Claim 1, characterised in that the said active phase corresponds to the formula (I) in which:

-   the symbol a represents a whole or fractional number up to 20 and is preferably between 1 and 10,
-   the symbol b represents a whole or fractional number up to 20 and is preferably between 1 and 10.

**3.** Process according to one of Claims 1 or 2, characterised in that the alkane is propane.

**4.** Process according to one of Claims 1 to 3, characterised in that the reaction is carried out in the presence of steam.

**5.** Process according to one of Claims 1 to 4, characterised in that the reaction temperature is between 300°C and 550°C and preferably between 350°C and 500°C.

6. Process according to one of Claims 1 to 5, characterised in that the total pressure is between 1 and 6 bar.

7. Process according to one of Claims 1 to 6, characterised in that the hourly volume rate is between 100 and 36,000 $h^{-1}$.

8. Process according to one of Claims 1 to 7, characterised in that, in the reactive gas (mixture of saturated hydrocarbon, ammonia and oxygen), the content of saturated hydrocarbon is between 5 and 70 %, the content of ammonia is between 3 and 50 % and the content of oxygen is between 3 and 45 %.

9. Process according to one of Claims 1 to 8, characterised in that the composition of the gaseous mixture is outside the explosive region.

10. Process according to one of Claims 1 to 9, characterised in that the solid catalyst contains, besides the said active phase, at least one inorganic oxide.

11. Process according to Claim 10, characterised in that the inorganic oxide is chosen from alumina, silica, silica/alumina, zirconia, cerite, magnesia, titanium oxide, niobium oxide and their mixtures.

12. Process according to one of Claims 1 to 9, characterised in that the solid catalyst contains, besides the said active phase, an inert support or a ceramic or metal substrate of monolithic type.

13. Process according to either of Claims 10 and 11, characterised in that the solid catalyst also contains an inert support or a ceramic or metal substrate of monolithic type.

14. Process according to either of Claims 10 and 11, characterised in that the active phase of the catalyst represents from 5 % to 100 % by weight of the weight of the said catalyst and preferably from 10 % to 50 % weight/weight.

15. Process according to either of Claims 12 and 13, characterised in that the active phase or the catalytic phase represents from 1 % to 50 % by weight of the weight of the catalyst and preferably from 5 % to 35 % weight/weight.

**Patentansprüche**

1. Verfahren zur Ammoxidation von Alkanen in der Dampfphase und in Anwesenheit eines festen Katalysators, der mindestens eine aktive Phase umfaßt, dadurch gekennzeichnet, daß die genannte aktive Phase der folgenden empirischen Formel (I):

$$V\,Sb_a\,M_b\,O_x \qquad\qquad (I)$$

entspricht, worin

- a eine ganze oder gebrochene Zahl von gleich oder höher als 1 darstellt,
- M ein Atom von Eisen und/oder Gallium und/oder Indium bedeutet,
- b eine ganze oder gebrochene Zahl von gleich oder höher als 0,5 darstellt,
- x eine ganze oder gebrochene Zahl bedeutet, die durch den Oxidationsgrad der anderen Elemente der aktiven Phase bestimmt wird.

2. Verfahren nach Anspruch 1, dadurch gekennzeichnet, daß die genannte aktive Phase der Formel (I) entspricht, worin

- das Symbol a eine ganze oder gebrochene Zahl von bis zu 20, vorzugsweise zwischen 1 und 10 darstellt,
- das Symbol b eine ganze oder gebrochene Zahl von bis zu 20, vorzugsweise zwischen 1 und 10 darstellt.

3. Verfahren nach einem der Ansprüche 1 oder 2, dadurch gekennzeichnet, daß das Alkan Propan ist.

**4.** Verfahren nach einem der Ansprüche 1 bis 3, dadurch gekennzeichnet, daß die Reaktion in Anwesenheit von Wasserdampf durchgeführt wird.

**5.** Verfahren nach einem der Ansprüche 1 bis 4, dadurch gekennzeichnet, daß die Temperatur der Reaktion zwischen 300 °C und 550 °C, vorzugsweise zwischen 350 °C und 500 °C liegt.

**6.** Verfahren nach einem der Ansprüche 1 bis 5, dadurch gekennzeichnet, daß der Gesamtdruck zwischen 1 und 6 bar liegt.

**7.** Verfahren nach einem der Ansprüche 1 bis 6, dadurch gekennzeichnet, daß die Stundenvolumen-Geschwindigkeit zwischen 100 und 36000 $h^{-1}$ liegt.

**8.** Verfahren nach einem der Ansprüche 1 bis 7, dadurch gekennzeichnet, daß in dem Reaktionsgas (Mischung von gesättigtem Kohlenwasserstoff, Ammoniak und Sauerstoff) der Gehalt an gesättigtem Kohlenwasserstoff zwischen 5 % und 70 %, der Gehalt an Ammoniak zwischen 3 % und 50 % und der Gehalt an Sauerstoff zwischen 3 % und 45 % liegt.

**9.** Verfahren nach einem der Ansprüche 1 bis 8, dadurch gekennzeichnet, daß die Zusammensetzung der Gasmischung außerhalb des Bereiches der Explosionsfähigkeit liegt.

**10.** Verfahren nach einem der Ansprüche 1 bis 9, dadurch gekennzeichnet, daß der feste Katalysator außer der genannten aktiven Phase mindestens ein mineralisches Oxid umfaßt.

**11.** Verfahren nach Anspruch 10, dadurch gekennzeichnet, daß das mineralische Oxid unter Aluminiumoxid, Siliciumdioxid, Silicium-Aluminiumoxid, Zirkoniumdioxid, Ceroxid, Magnesiumoxid, Titanoxid, Niobiumoxid und ihren Mischungen ausgewählt wird.

**12.** Verfahren nach einem der Ansprüche 1 bis 9, dadurch gekennzeichnet, daß der feste Katalysator außer der genannten aktiven Phase einen inerten Träger oder ein keramisches oder metallisches Substrat vom monolithischen Typ umfaßt.

**13.** Verfahren nach einem der Ansprüche 10 oder 11, dadurch gekennzeichnet, daß der feste Katalysator ebenfalls einen inerten Träger oder ein keramisches oder metallisches Substrat vom monolithischen Typ umfaßt.

**14.** Verfahren nach einem der Ansprüche 10 oder 11, dadurch gekennzeichnet, daß die aktive Phase des Katalysators 5 Gew.-% bis 100 Gew.-% des Gewichtes des genannten Katalysators und vorzugsweise 10 Gew.-% bis 50 Gew.-% darstellt.

**15.** Verfahren nach einem der Ansprüche 12 oder 13, dadurch gekennzeichnet, daß die aktive Phase oder die katalytische Phase 1 Gew.-% bis 50 Gew.-% des Gewichtes des Katalysators und vorzugsweise 3 Gew.-% bis 35 Gew.-% darstellt.

FIG.1

FIG.2